# FASCICULE DE BREVET EUROPEEN

(11) **EP 0 493 223 B1**
(45) Date de publication et mention de la délivrance du brevet: **30.11.1994**
(21) Numéro de dépôt: 91403484.8
(22) Date de dépôt: 20.12.1991
(51) Int. Cl.: A61K 7/48, A61K 35/78

(54) **Compositions cosmétiques à base d'extraits végétaux**
Kosmetische Zusammensetzung auf der Basis von Pflanzenextrakten
Cosmetic compositions based on vegetable extracts

(30) Priorité: 21.12.1990 FR 9016159
(43) Date de publication de la demande: 01.07.1992
(73) Titulaire: SANOFI, 75008 Paris (FR)
(72) Inventeur: Courregelongue, Jean, F-31120 Portet-sur-Garonne (FR); Dumont, Pascale, F-31100 Toulouse (FR); Sabadie, Michel, F-27500 Bernay (FR)
(74) Mandataire: Gillard, Marie-Louise

(56) Documents cités:
- FR-A- 2 563 109
- FR-A- 2 578 422
- CHEMICAL ABSTRACTS, vol. 102, no. 19, 13 Mai 1985, Columbus, Ohio, US; abstract no. 163722A, BISWANATH DASGUPTA ET AL.: 'Major alkaloid and flavonoid of Premna integrifolia' page 355 ;

## Description

La présente invention concerne des compositions cosmétiques pour le traitement et le soin de la peau contenant des extraits de plantes orientales et plus particulièrement de plantes birmanes.

Les propriétés odoriférantes de certains bois de la Birmanie et particulièrement de Thanat-Kha (Limonia acidissima) et de Kalamet (Mansonia gagei) sont bien connues.

Il est aussi connu qu'en Birmanie et en Thaïlande, le bois des arbres morts de mort naturelle peuvent être utilisés en tant que cosmétiques: ils sont traditionnellement preparés en broyant l'écorce ou le bois sur marbre humidifié et en mélangent la substance ainsi obtenue avec une petite quantité d'eau avant de l'appliquer directement sur la peau.

Des produits cosmétiques pour la protection de la peau à base de poudres ou d'extraits de Thanat-kha sont décrits dans la demande de brevet Japonais JP 83-65209.

On sait également que par extraction des feuilles de Premna integrifolia on peut obtenir la lutéoline (*Chemical Abstracts, 1985, 102, n° 163722a*), un flavonoïde que l'on peut extraire d'un grand nombre de plantes, et dont les propriétés dépigmentantes ont été décrites dans FR-A-2 578 422.

On a maintenant trouvé qu'on peut obtenir, à partir du bois de Kalamet (Mansonia gagei) ou de Taung-taunggi (Premna integrifolia), des extraits qui ont des propriétés cosmétiques très importantes et très spécifiques et qui se prêtent bien à la préparation de compositions cosmétiques pour le soin de la peau.

Plus particulièrement on a trouvé que, à partir de ces bois on peut obtenir des extraits ayant la propriété d'inhiber la collagénase et la hyaluronidase.

On a également trouvé que des extraits obtenus à partir du Kalamet ou du Taung-taunggi sont capables de capturer les radicaux libres et d'augmenter l'activité de la tyrosinase.

Ces deux dernières proprietés, sont particulièrement surprenantes car elles semblent spécifiques des extraits de Kalamet et de Taung-taunggi. Par exemple, les extraits de Thanat-kha sont inactifs sur les radicaux libres et sur la tyrosinase.

Le Kalamet (Mansonia gagei) est un arbre de la famille des Sterculiaceae, découvert dans le Tenasserim du Sud en Birmanie par F.B. Manson. Il peut pousser jusqu'à 12 m de hauteur et avoir une circonférence d'environ 1 m. Il a une distribution tout-à-fait locale, étant essentiellement confiné dans les bassins des fleuves Sarawa et Kalamet.

Le Taung-taunggi (Premna integrifolia) est un arbuste de la famille des Verbenaceae distribué sur le littoral de Tenasserim. Il est caracterisé par un bois assez dur de couleur brun velouté.

Pour la préparation des extraits, on coupe le bois entier ou la partie interne du bois de Kalamet ou de Taung-taunggi en très petits morceaux, ou on peut aussi le pulvériser. On traite ensuite le substrat avec un solvant ou un mélange de solvants d'extraction convenablement choisis et, après quelques heures, on filtre et on évapore à sec la solution ainsi obtenue.

Si on le désire, on répète la procédure d'extraction, à partir du résidu ainsi obtenu, avec le même solvant ou mélange de solvants ou avec un solvant ou un mélange de solvants différents.

Les solvants organiques qui peuvent être utilisés de manière avantageuse, tels quels ou sous forme de mélanges, sont les alcanols inférieurs, par exemple le méthanol, l'éthanol et l'isopropanol, les glycols tels que l'éthylène-glycol et le propylène-glycol, les dérivés mono-éthers correspondants tels que le 2-méthoxyéthanol et le 2-éthoxy-éthanol, les éthers tels que l'éther éthylique, l'éther isopropylique ou le diméthoxy-éthane, les hydrocarbures aliphatiques halogénés tels que le chloroforme, le chlorure de méthylène, et le 1,1,1-trichloroéthylène, les cétones, telles que l'acétone, les hydrocarbures aromatiques tels que le toluène. etc.

Ces solvants ou mélanges de solvants, peuvent aussi contenir des proportions variables d'eau, selon leur miscibilité.

Les solvants et les mélanges de solvants particulièrement avantageux sont les alcanols inférieurs tel que le méthanol et l'éthanol, les mélanges d'un alcanol inférieur, tel que le méthanol ou l'éthanol, ou d'un glycol inférieur tel que l'éthylène-glycol ou le propylène-glycol avec l'eau, en proportions, exprimées en volume, comprises entre 30:70 et 70:30; les autres mélanges préférés sont les mélanges d'un alcanol ou d'un glycol inférieur tels que défini ci-dessus avec un hydrocarbure aliphatique halogéné tel que le chloroforme, le chlorure de méthylène ou le 1,1,1-trichloroéthane, en proportions qui varient aussi selon leurs miscibilités respectives.

En ce qui concerne le rapport entre le volume de substrat et le volume de solvant ou de mélange de solvants d'extraction (système solvant d'extraction) à utiliser, il est en général compris entre 1:3 et 1:20, et de préférence entre 1:7 et 1:15. On peut utiliser aussi des volumes de système solvant plus grands mais l'augmentation des coûts de production ainsi entrainée ne serait pas compensée par le gain de rendement d'extraction.

Après quelques heures, en général entre 8 et 16 heures, on filtre à température ambiante et l'évaporation des solvants sous vide conduit à un résidu.

Ce résidu, comme on a déjà indiqué, peut-être soumis à une autre extraction suivie par évaporation du solvant ou mélange de solvants sous vide.

Si, pour l'extraction, on utilise des solvants qui sont, même à l'état de traces, toxiques ou inadaptés pour une utilisation cosmétique, on peut purifier les extraits ainsi obtenus par traitement, éventuellement répété, avec une petite quantité de méthanol ou d'éthanol suivi par évaporation du solvant de rinçage.

A titre d'exemple, selon l'invention, on obtient aisément des extraits par le procédé général indiqué ci-dessus en utilisant en tant que système solvant un alcanol inférieur tel que le méthanol ou l'éthanol, un mélange méthanol/eau 50/50 (v/v) ou un mélange méthanol/chlorure de méthylène 50/50 (v/v), avec un rapport substrat/solvant de 1/10, en laissant l'extraction se poursuivre pendant 12 heures, en éliminant la partie insoluble par filtration à la température ambiante et en évaporant le ou les solvants sous pression réduite (10 mm Hg équivalents à environ 1300 Pa). On peut ensuite soumettre l'extrait ainsi obtenu à une autre extraction selon les indications générales données ci-dessus.

Les activités des extraits ainsi obtenus ont été mises en évidence par des méthodologies derivées des techniques classiques normalement utilisées en biochimie, qui sont depuis quelques années bien connues et largement employées en cosmétologie.

Plus particulièrement, ces extraits ont été soumis aux tests de l'inhibition de la collagénase, de la capture des radicaux libres, de l'activation de la tyrosinase et de l'inhibition de la hyaluronidase.

Dans le premier de ces tests, les extraits ainsi obtenus ont montré une remarquable activité dans l'inhibition de la collagénase, enzyme protéolytique qui entraîne la dégradation du collagène, une des deux principales proteines structurales de la peau. Son rôle dans le maintien de l'intégrité de la peau, notamment dans le processus de vieillissement, est primordial.

Les extraits de l'invention se sont montrés aussi très actifs en tant que capteurs des radicaux libres.

Les radicaux libres, qui sont générés au niveau de la peau par les diverses aggressions physiques et chimiques de l'environnement, et principalement par les rayons U.V., sont très dangereux (*B. Halliwell et J.M.C. Gutteridge - Free Radicals in Biology and Medicine, Oxford : Clarendon Press; 1985:346*). Ils oxydent les lipides de la membrane cellulaire, détruisent les protéines structurales, dénaturent les enzymes et peuvent même arriver à modifier le code génétique. Les extraits de l'invention, très actifs dans la capture des radicaux libres dès leur formation, peuvent donc être convenablement utilisés dans une composition cosmétique, pour atténuer les effets néfastes que les agents agressifs, notamment le soleil (érythème, viellissement accéléré, etc.), ont sur la peau.

Des extraits de l'invention sont aussi activateurs de la tyrosinase, enzyme qui joue un rôle fondamental dans la synthèse des eumélanines à partir de la tyrosine et peuvent donc être employés dans des compositions cosmétiques pour faciliter le bronzage.

On peut donc envisager l'utilisation des extraits de la présente invention dans le domaine cosmétique, pour retarder ou combattre le vieillissement de la peau et la dégénérescence tissulaire, et pour faciliter le bronzage. Dans ce dernier cas, les propriétés antiradicalaires sont extrêmement utiles car les rayons du soleil facilitent la formation des radicaux libres.

Pour l'utilisation cosmétique, les extraits selon la présente invention peuvent être formulés sous forme de crèmes, gels, lotions, émulsions, solutions, onguents, etc., qui les contiennent en quantités comprises entre 0,001 et 10 %, de préférence entre 0,005 et 5 % en poids. Quand on utilise le bois de Taung-taunggi, les extraits de l'invention sont en général utilisés en quantités inférieures à 0,1 %, de préférence inférieures à 0,05 %, et notamment entre 0,005 et 0,02 % en poids, à cause de la couleur qui peut être donnée par l'extrait au produit fini.

Pour la préparation de ces compositions cosmétiques, les extraits sont mélangés aux excipients généralement employés dans la technique cosmétique tels que, par exemple, les matières grasses d'origine animale, les huiles végétales, les acides gras saturés ou insaturés, les alcools, les ésters d'acides gras et d'alcool gras, les glycols polyalkyléniques, les cires, la vaseline, les polyéthers et peuvent être associés à l'eau et à des agents gelifiants lorsqu'ils sont compatibles.

Pour une utilisation plus aisée des extraits de l'invention dans la préparation de compositions cosmétiques, on les mélange aux excipients et additifs sous forme de solution dans un solvant ou mélange de solvants approprié pour l'utilisation cosmétique prévue. A titre d'exemple, on peut avantageusement utiliser ces extraits sous forme de solutions dans des mélanges glycol/eau, selon la solubilité des extraits mêmes.

La forme de la composition cosmétique de la présente invention pourrait être une crème dans laquelle l'extrait est associé aux excipients couramment utilisés dans la cosmétologie et qui seraient compatibles avec celui-ci tel quel la lanoline et ses derivés.

Les compositions cosmétiques peuvent être également préséntées sous forme de gel dans les excipients appropriés tels que les esters de cellulose ou d'autres agents gélifiants, tels que le carbopol, la gomme guar, etc.

Les compositions cosmétiques suivant l'invention peuvent aussi prendre la forme de lotion ou solution dans laquelle l'extrait est dissout ou microdispersé dans une microémulsion.

La forme des compositions cosmétiques suivant l'invention peut donc être une microdispersion de l'extrait dans un liquide contenant de l'eau, ainsi qu'un ou plusieurs agents tensioactifs. Ces dispersions présentent les caractères des microémulsions et ont pratiquement l'aspect de solutions vraies. Elles sont de préférence préparées extemporanément.

Ces microémulsions jouissent d'une bonne stabilité et peuvent être conservées pendant le temps nécessaire pour l'utilisation à des températures comprises entre 0 et 60° C sans qu'il y ait sédimentation des constituants ou séparation des phases.

Les tensioactifs de la composition sont choisi parmi les agents surfactants utilisables en cosmétologie. On peut indiquer à titre d'exemples non limitatifs: les esters de sorbitol et leurs dérivés polyoxyéthylénés, les huiles de ricin (hydrogénées ou non) polyoxyéthylénées, les copolymères séquencés oxyde d'éthylène/oxyde de propylène, les acides gras polyoxyéthylénés, les alcools gras polyoxyéthylénées, le laurylsulfate de sodium, le dioctylsulfosuccinate de sodium, les lécithines d'oeuf ou de soja.

Les compositions cosmétiques de l'invention peuvent aussi contenir des additifs ou des adjuvants usuels en cosmétologie, comme par exemple des agents antibactériens ou des parfums.

Les compositions cosmétiques de la présente invention peuvent aussi comprendre d'autres principes actifs complémentaires choisis pour leur action par exemple sur la nutrition cellulaire, la respiration cellulaire, l'hydratation et la régénération cellulaire, ainsi que d'autres principes actifs ayant le même type d'action de protection et de défense cellulaire de la peau.

Lorsque les compositions cosmétiques de la présente invention contiennent des principes actifs complémentaires, ceux-ci sont généralement présents dans la composition à une concentration suffisamment élevée pour qu'ils puissent exercer leur activité.

Les principes actifs complémentaires sont par exemple des substances hydratantes telles que l'acide hyaluronique, ou le sel de sodium de l'acide pyrrolidonecarboxylique, des agents protecteurs tels que le panthénol, une substance vitaminique protectrice, des facteurs de croissance cellulaire tels que les extraits de rate bovine ou les extraits de levure, etc.

Les compositions cosmétiques de la présente invention sont de préférence à utiliser quotidiennement en les appliquant une ou plusieurs fois par jour, selon l'état de la peau.

Les compositions cosmétiques de la présente invention sont très bien tolérées, elles ne présentent aucune phototoxicité et leur application sur la peau pour des périodes de temps prolongées n'implique aucun effet systémique. En général, après trente jours d'application, on note une amélioration de l'aspect de la peau et un ralentissement dans la formation des rides.

Les compositions cosmétiques de la présente invention peuvent aussi bien être formulées comme produits pour le maquillage, tels que par exemple, fond de teint, poudre, fard à paupières, ou rouge à lèvres.

Pour la préparation des fonds de teint on peut mélanger un extrait selon la présente invention à une base de lotion ou de solution contenant des quantités convenables d'agents épaississants, des pigments et des parfums.

La poudre, ainsi que le fard à paupières, peut être obtenue, selon les méthodes conventionnelles en mélangeant l'extrait à une base pour poudre ou pour fard, qui généralement contient du talc, des carbonates, des oxydes, des quantités convenables de parfums et de pigments, de préférence organiques, et éventuellement des agents liants.

Pour la préparation des rouges à lèvres, l'extrait est mélangé avec une base huile-cire assez pâteuse pour former un baton, contenant les pigments appropriés.

Les exemples suivants illustrent l'invention sans toutefois la limiter.

### EXEMPLE 1

On coupe du bois de Kalamet en copeaux que l'on traite par un mélange méthanol/eau 50/50 (v/v) en raison de 10 ml de solvant par gramme de copeaux.
Après 12 heures à température ambiante, on filtre et on évapore le filtrat sous pression réduite (environ 1300 Pa) jusqu'à en obtenir un résidu pâteux. Ce résidu est repris par 2 ml de méthanol. La solution ainsi obtenue est soumise aux tests cosmétiques indiqués ci-dessus. Ainsi on a mis en évidence une activité inhibitrice de la collagénase, une activité antiradicalaire et une activité inhibitrice de la hyaluronidase.

### EXEMPLES 2

En suivant la procédure de l'Exemple 1 en utilisant de l'éthanol à 95% comme solvant d'extraction on obtient, à partir de 250 gr de copeaux, un extrait sec de 3,1 g qui, après mise en solution méthanolique, montre une activité inhibitrice de la collagénase, une activité antiradicalaire et une activité inhibitrice de la hyaluronidase.

### EXEMPLE 3

En suivant le mode opératoire décrit dans l'Exemple 1 et en utilisant, en tant que solvant d'extraction, un mélange méthanol/chlorure de méthylène 50/50 (v/v), on obtient un extrait qui, sous forme de solution méthanolique montre une activité inhibitrice de la collagénase et une activité antiradicalaire très intéressantes.

### EXEMPLE 4

On suit la procédure de l'Exemple 1 à partir d'une poudre de Kalamet obtenue à partir du bois brut par broyage, et on obtient un extrait qui possède l'activité inhibitrice de la collagénase ainsi que l'activité antiradicalaire de l'extrait de l'Exemple 1 et montre aussi une activité de stimulation de la tyrosinase.

### EXEMPLE 5

En suivant la procédure de l'Exemple 3 mais à partir de la poudre de Kalamet utilisée dans l'Exemple 4 on obtient un extrait qui montre les mêmes activités de l'extrait de l'Exemple 3.

### EXEMPLE 6

En opérant comme décrit dans l'Exemple 1 à partir du bois de Taung-taunggi pulvérisé, on obtient un extrait très actif dans l'inhibition de la collagénase, la capture des radicaux libres et l'activation de la tyrosinase.

### EXEMPLE 7

A partir du bois de Taung-taunggi pulvérisé, en utilisant la procédure d'extraction décrite dans l'Exemple 3, on obtient un extrait qui a les mêmes activités que l'extrait obtenu dans l'Exemple 6.

### EXEMPLE 8

A partir d'une poudre de Taung-taunggi obtenue à partir du bois brut par broyage, en utilisant la procédure d'extraction décrite dans l'Exemple 2 et en évaporant ensuite le méthanol, on obtient un extrait qui a les mêmes activités que l'extrait obtenu dans l'Exemple 6.

### EXEMPLES 9-15

On prépare les compositions suivantes qui sont utilisées comme indiqué dans chaque exemple.

### EXEMPLE 9

| ***Gel hydratant*** | |
|---|---|
| Extrait de l'Exemple 1 | 0,50 g |
| Carbopol 940 | 0,20 g |
| Polyéthylène glycol | 3,00 g |
| Conservateurs | 0,50 g |
| Butylène glycol | 5,00 g |
| Laurate de sorbitan éthoxylé | 0,50 g |
| Parfum | 0,20 g |
| Triéthanolamine | 0,25 g |
| Eau déminéralisée | Q.S.P. 100 g |

### EXEMPLE 10

| ***Crème de nuit*** | |
|---|---|
| Extrait de l'Exemple 4 | 1,00 g |
| Alcool cétylique | 2,00 g |
| Stéarine | 2,50 g |
| Monostéarate de glycérol | 5,00 g |
| Palmitate d'isopropyle | 5,00 g |
| Huile végétale | 3,00 g |
| Huile minérale | 2,00 g |
| Perhydrosqualène | 2,00 g |
| Huile de silicone | 1,00 g |
| Beurre de karité | 2,00 g |
| Palmitate d'éthyl-2-hexyle | 5,00 g |
| Triéthanolamine | 5,50 g |
| Conservateurs | 0,50 g |
| Butylène glycol | 5,00 g |
| EDTA tétrasodique | 0,30 g |
| Parfum | 0,31 g |
| Antioxydant | 0,10 g |
| Eau déminéralisée | Q.S.P. 100 g |

### EXEMPLE 11

| ***Sérum hydratant*** | |
|---|---|
| Extrait de l'Exemple 8 | 0,01 g |
| Hydroxypropylméthylcellulose | 0,30 g |
| Propylène glycol | 5,00 g |
| Glycérine | 5,00 g |
| Alcool oleique éthoxylé | 0,50 g |
| Parfum | 0,30 g |
| EDTA tétrasodique | 0,10 g |
| Conservateurs | 0,50 g |
| Albumine bovine | 5,00 g |
| Eau déminéralisée | Q.S.P. 100 g |

### EXEMPLE 12

| ***Base fluide de maquillage*** | |
|---|---|
| Extrait de l'Exemple 8 | 0,005 g |
| Stérols de soja éthoxylés | 4,00 g |
| Stérols de soja | 0,50 g |
| Monostéarate de glycérol | 1,00 g |
| Huile végétale | 1,50 g |
| Palmitate d'éthyl 2-hexyle | 4,00 g |
| Alcool cétylique | 0,50 g |
| Triglycérides capriques/capryliques | 1,50 g |
| Huile de silicone | 1,00 g |
| Huile minérale | 1,80 g |
| Alcools de lanoline | 0,20 g |
| Dipélargonate de propylène glycol | 3,00 g |
| Lécithine | 1,00 g |
| Conservateurs | 0,50 g |
| Butylène glycol | 5,00 g |
| Carbopol | 0,20 g |
| Triéthanolamine | 0,20 g |
| EDTA tétrasodique | 0,10 g |
| Antioxydant | 0,01 g |
| Parfum | 0,30 g |
| Eau déminéralisée | Q.S.P. 100 g |

### EXEMPLE 13

| ***Crème de jour protectric*e** | |
|---|---|
| Extrait de l'Exemple 6 | 0,02 g |
| Monostéarate de sorbitan éthoxylé | 2,60 g |
| Huile de silicone | 1,00 g |
| Alcool cétylique | 2,00 g |
| Huile minérale | 3,00 g |
| Alcools de lanoline | 1,00 g |
| Perhydrosqualène | 1,00 g |
| Monostéarate de sorbitan | 2,40 g |
| Palmitate de cétyle | 3,00 g |
| Palmitate d'isopropyle | 4,00 g |
| Filtre UVA - UVB | 2,00 g |
| EDTA tétrasodique | 0,10 g |
| Carbopol | 0,30 g |
| Triéthanolamine | 0,30 g |
| Antioxydant | 0,01 g |
| Conservateurs | 0,50 g |
| Parfum | 0,30 g |
| Butylène glycol | 5,00 g |
| Eau déminéralisée | Q.S.P. 100 g |

### EXEMPLE 14

| ***Microémulsions hydratantes*** | |
|---|---|
| Extrait de l'Exemple 3 | 0,10 g |
| Hydroxystéarate de polyéthylène glycol 600 | 1,00 g |
| Triglycérides 7-8 atomes de carbone polyoxyéthylénés | 0,25 g |
| Polyéthylène glycol-7 glycéryl cocoate | 0,20 g |
| Diméthicone copolyol | 0,05 g |
| Propylène glycol | 12,50 g |
| Ethanol | 12,50 g |
| Carbopol | 0,40 g |
| Triéthanolamine | 0,40 g |
| Parfum | 0,30 g |
| Colorants | Q.S. |
| Conservateurs | 0,50 g |
| Eau déminéralisée | Q.S.P. 100 g |

### EXEMPLE 15

| ***Fond de teint hydratant*** | |
|---|---|
| Extrait de l'Exemple 2 | 5,00 g |
| EDTA tétrasodique | 0,10 g |
| Carboxyméthylcellulose | 0,20 g |
| Silicate d'aluminium et de magnésium | 1,00 g |
| Laurate de sorbitan éthoxylé | 1,00 g |
| Propylène glycol | 5,00 g |
| Oxyde de titane | 2,00 g |
| Talc | 1,00 g |
| Pigments | 1,00 g |
| Triéthanolamine | 0,50 g |
| Conservateurs | 0,50 g |
| Alcool cétylique | 1,00 g |
| Alcool de lanoline | 3,00 g |
| Acide stéarique | 1,80 g |
| Monostéarate de propylène glycol | 3,00 g |
| Palmitate d'isopropyle | 8,00 g |
| Huile végétale | 2,00 g |
| Antioxydant | 0,05 g |
| Parfum | 0,30 g |
| Eau déminéralisée | Q.S.P. 100 g |

## Revendications

1. Une composition cosmétique pour le traitement et le soin de la peau caractérisée en ce qu'elle comprend un extrait obtenu du bois de Kalamet (Mansonia gagei) ou de Taung-taunggi (Premna-integrifolia) en combinaison avec un excipient ou un diluant acceptable du point de vue cosmétique et dermatologique.

2. La composition cosmétique de la revendication 1 caractérisée en ce que l'extrait de Kalamet ou de Taung-taunggi est présent en une quantité comprise entre 0,001 et 10% en poids.

3. La composition cosmétique de la revendication 2 caractérisée en ce que l'extrait est présent en une quantité comprise entre 0,005 et 5% en poids.

4. La composition cosmétique de la revendication 3 caractérisée en ce qu'elle comprend un extrait obtenu du bois de Taung-taunggi en quantité comprise entre 0,005 et 0,02% en poids.

5. La composition cosmétique d'une quelconque des revendications précédentes de 1 à 4, caractérisée en ce que l'extrait de Kalamet ou de Taung-taunggi est obtenu à partir du bois entier ou de la partie interne du bois par extraction avec un solvant d'extraction approprié, filtration et évaporation à sec du filtrat, cette procédure d'extraction étant éventuellement repétée sur l'extrait ainsi obtenu.

6. La composition cosmétique de la revendication 5 caractérisée en ce que le solvant d'extration est choisi parmi les alcanols inférieurs, les glycols inférieurs, les glycols monoéthers, les éthers alkyliques, les hydrocarbures aliphatiques halogénés, les cétones, les hydrocarbures aromatiques et leur mélanges, contenant éventuellement des proportions variables d'eau.

7. La composition de la revendication 6 caractérisée en ce que le solvant d'extraction est choisi parmi le méthanol, l'éthanol, les mélanges méthanol/eau et les mélanges méthanol/chlorure de méthylène.

8. Utilisation de la composition d'une quelconque des revendications de 1 à 7 pour retarder ou combattre le vieillissement de la peau et la dégénérescence tissulaire et pour faciliter le bronzage.

9. Procédé pour la préparation d'une composition cosmétique selon l'une quelconque des revendications 1 à 7 caractérisé en ce qu'on mélange un extrait obtenu du bois de Kalamet (Mansonia gagei) ou de Taung-taunggi (Premna integrifolia) avec au moins un excipient ou un diluant acceptable du point de vue cosmétique et dermatologique.

## Claims

1. A cosmetic composition for skin care and treatment, characterised in that it comprises an extract obtained from Kalamet (Mansonia gagei) or Taung-taunggi (Premna integrifolia) wood in admixture with a cosmetically and dermatologically acceptable excipient or diluent.

2. The cosmetic composition of claim 1, characterised in that it comprises between 0.001 and 10% by weight of Kalamet or Taung-taunggi wood extract.

3. The cosmetic composition of claim 2, characterised in that it comprises between 0.005 and 5% by weight of Kalamet or Taung-taunggi wood extract.

4. The cosmetic composition of claim 3, characterised in that it comprises between 0.005 and 0.02% by weight of Taung-taunggi wood extract.

5. The cosmetic composition of any one of the preceding claims 1 to 4, characterised in that the Kalamet or Taung-taunggi extract is obtained from the whole wood or the internal part thereof by extraction with a suitable extraction solvent, filtration and dry-evaporation of the filtrate, this extraction process being optionally repeated on the extract thus obtained.

6. The cosmetic composition of claim 5, characterised in that the extraction solvent is selected among the lower alkanols, lower glycols, glycol monoethers, alkyl ethers, halogenated aliphatic hydrocarbons, ketones, aromatic hydrocarbons and mixtures thereof, optionally containing varying proportions of water.

7. The cosmetic composition of claim 6, characterised in that the extraction solvent is selected among the methanol, ethanol, methanol/water mixtures, and methanol/methylene chloride mixtures.

8. Use of the composition of any one of claims 1 to 7 for slowing down or treating skin ageing and tissue degenerescence and to facilitate suntanning.

9. Method for preparing a cosmetic composition according to any one of claims 1 to 7, characterised in that it consists in mixing an extract obtained from Kalamet (Mansonia gagei) or Taung-taunggi (Premna integrifolia) wood in admixture with a cosmetically and dermatologically acceptable excipient or diluent.

## Patentansprüche

1. Eine kosmetische Zusammensetzung für die Behandlung und Pflege der Haut, dadurch gekennzeichnet, daß sie einen Extrakt aus Kalamet-Holz (Mansonia gagei) oder Taung-taunggi-Holz (Premna-integrifolia) in Verbindung mit einem kosmetisch und dermatologisch geeigneten Bindungs- oder Verdünnungsmittel enthält.

2. Die kosmetische Zusammensetzung des Anspruchs 1, dadurch gekennzeichnet, daß der Kalamet- oder Taung-taunggi-Extrakt in einer Menge zwischen 0,001 und 10 Gew.-% vorhanden ist.

3. Die kosmetische Zusammensetzung des Anspruchs 2, dadurch gekennzeichnet, daß der Extrakt in einer Menge zwischen 0,005 und 5 Gew.-% vorhanden ist.

4. Die kosmetische Zusammensetzung des Anspruchs 3, dadurch gekennzeichnet, daß sie einen Extrakt aus Taung-taunggi-Holz in einer Menge zwischen 0,005 und 0,02 Gew.-% enthält.

5. Die kosmetische Zusammensetzung eines der vorstehenden Ansprüche 1 bis 4, dadurch gekennzeichnet, daß der Kalamet- oder Taung-taunggi-Extrakt aus dem ganzen Holz oder dem Inneren des Holzes gewonnen wird, durch Extraktion mittels eines geeigneten Extraktionslösungsmittels, Filtrierung und Eindampfung des Filtrats bis zur Trockene, wobei dieser Extraktionsvorgang eventuell an dem so erhaltenen Extrakt wiederholt wird.

6. Die kosmetische Zusammensetzung des Anspruchs 5, dadurch gekennzeichnet, daß das Extraktionslösemittel aus den niedrigen Alkanolen, den niedrigen Glykolen, den Monoetherglykolen, den Alkylethern, den aliphatischen Halogenkohlenwasserstoffen, den Ketonen, den aromatischen Kohlenwasserstoffen und ihren Mischungen, die eventuell variable Wasseranteile enthalten, ausgewählt ist.

7. Die Zusammensetzung des Anspruchs 6, dadurch gekennzeichnet, daß das Extraktionslösemittel aus Methanol, Ethanol, Methanol-Wasser-Gemischen und Methanol-Methylenchlorid-Gemischen ausgewählt wird.

8. Verwendung der Zusammensetzung eines der Ansprüche 1 bis 7 zur Verzögerung oder Bekämpfung der Alterung der Haut und der Degeneration des Gewebes und Erleichterung der Bräunung.

9. Verfahren zur Herstellung einer Zusammensetzung nach einem der Ansprüche 1 bis 7, dadurch gekennzeichnet, daß man einen aus dem Kalamet- (Mansonia gagei) oder Taung-taunggi- (Premna integrifolia) -Holz erhaltenen Extrakt mit mindestens einem kosmetisch und dermatologisch geeigneten Bindungs- oder Verdünnungsmittel mischt.
